# EUROPEAN PATENT SPECIFICATION

(11) **EP 3 034 052 B1**
(45) Date of publication and mention of the grant of the patent: **02.08.2017**
(21) Application number: 15199983.6
(22) Date of filing: 15.12.2015
(51) Int. Cl.: A61F 5/37, A61F 5/048, A61F 5/01, A61F 5/058

(54) **ELASTIC SHOULDER BRACE**
ELASTISCHE SCHULTERORTHESE
ORTHESE D'EPAULE ÉLASTIQUE

(30) Priority: 17.12.2014 IT MI20142164
(43) Date of publication of application: 22.06.2016
(73) Proprietor: Tenortho S.r.l. Unipersonale, 20853 Biassono (Monza Brianza) (IT)
(72) Inventor: TENTORIO, Alessio, 20052 Monza (Monza Brianza) (IT)
(74) Representative: Vittorangeli, Lucia

(56) References cited:
- US-A- 4 550 724
- US-A- 5 628 725
- US-A- 5 857 990
- US-A1- 2004 193 086
- US-A1- 2009 133 181

## Description

The present invention relates to an elastic brace for application on the joints of patients affected by joint diseases.

More specifically, the present invention can be particularly used for treating shoulder joint traumas.

As is known, elastic braces for joints have a flexible or semi-rigid structure, adapted to provide a support able to limit the movement of the injured joint.

Generally, in the case of an elbow, elastic braces are split into two main groups, each one dedicated to a particular action on the joint.

A first type of elastic brace is used in the sports field following anterior dislocation and sub dislocation of the humeral head. Such braces comprise a main portion made of elasticated fabric to be worn by the user so as to be wrapped around the area corresponding to the deltoid (shoulder).

The main portion has a tubular end adapted to be wrapped around the humerus. Such end may have a closed section to be fitted onto the arm, or have an open band to be wrapped around and anchored to the humerus by means of special belts or Velcro® fastenings.

The main portion also has an opposite strip to the tubular end adapted to be wrapped around the bust of the user so as to determine a traction action of the main portion towards the user's body.

The strip is generally adjusted through traction and anchoring systems, for example using Velcro® to adapt the tensile force to the size of the user.

In some known solutions, two strips may also be provided, respectively passing below the arm pit and onto the opposite collarbone to the injured joint.

The braces described above act on the joint by actuating a stabilisation and immobilisation action on the joint which, as described above, is mainly used following anterior dislocations and sub dislocations of the humeral head.

A second type of elastic brace is actually used for treating problems deriving from hemiplegia and/or hemiparesis, diseases which cause a gradual loss of motor activity. This leads to significant disadvantages both for the circulatory system and for the nerve plexuses, since both are subjected to continuous tensile stress in the downwards direction exerted by the paralysed limb.

Braces used for pathological problems differ from the first type (braces mainly for sports use) since the structure is more rigid acting consistently on the traction action of the arm towards the shoulder joint. For this purpose, the tubular end is anchored to a large part of the arm, extending in some solutions also to the part of the forearm proximal to the elbow. Furthermore, some solutions of this type envisage the presence of elastic strips that are also coupled to the terminal portion of the tubular end so as to constrain the arm better, and support it, preventing tensile stress in the downwards direction. Such braces are, for example, disclosed in US 5,857,990, which is regarded as the closest prior art, as well in US 5,628,725, and US 4,550,724. However, the elastic braces described above have significant disadvantages and limits to application.

In the first place, it must be considered that the braces are not versatile since they can be used for a specific purpose which may be that of containing the limb (for sports use), or retracting the arm (for therapeutic use) towards the shoulder.

Consequently, if the user needs to provide for both purposes, it is necessary to have two different braces, with the consequent disadvantages in terms of practicality, costs and time for the application of each brace.

In fact, within this context it is considered that an initial dislocation or sub dislocation of the humeral head, which therefore requires simple immobilisation of the limb, can degenerate into a pathology and lead to the need to support the arm.

In this case, the user must therefore initially provide for a brace for sports use and subsequently replace this sports brace with one for pathological use with the consequent disadvantages in terms of costs.

A further drawback of the braces of the type described above comes from the poor adaptability to the different injuries that the joint may have and which can therefore lead to one or more pathological conditions or not. In fact, elastic braces, even if they are generally adjustable according to the size of the joint (and the body of the user) are not adaptable according to the mechanical action, which may be more or less intense, to be actuated on the arm.

It should also be considered that, still in the case of pathologies, the injury to the joint may improve or decline with the consequent need to adjust the tensile force of the arm and the precise position in which such a force is actuated.

In this context, the technical objective underlying the present invention is to provide an elastic brace which obviates the drawbacks in the prior art as described above.

In particular, an object of the present invention is to provide an elastic brace that is versatile and able to implement on the joint both a stabilisation and immobilisation action typically used following dislocations, and a traction action of the arm in order to treat pathologies that cause a gradual loss of motor activity.

A further object of the present invention is to provide an elastic brace that is structurally simple, inexpensive, comfortable and easy to put on by the actual user.

It is also an object of the present invention to provide an elastic brace that allows intervention to be provided in a differentiated way on the joint according to the type and severity of the pathology to be treated.

The stated technical task and specified objects are substantially achieved by an elastic brace comprising the technical features disclosed in one or more of the appended claims.

Further characteristics and advantages of the present invention will become more apparent from the approximate, and thus non-limiting, description of a preferred, but not exclusive, embodiment of an elastic brace as illustrated in the accompanying drawings, wherein:
- figure 1 is a perspective and exploded view of an elastic brace according to the present invention applied to a shoulder;
- figure 2 is a perspective view of the elastic brace of figure 1 in a first operating condition;
- figure 3 is a perspective view of the elastic brace of figure 1 in a second operating condition;
- figures 4a to 4d show perspective views of respective operating sequences for the application of the elastic brace to the shoulder joint of a user;
- figure 5 shows a plan view of the elastic brace in a respective non-use condition wherein it is not applied to the joint; and
- figure 5a shows a plan view of a detail of the elastic brace in accordance with a further embodiment.

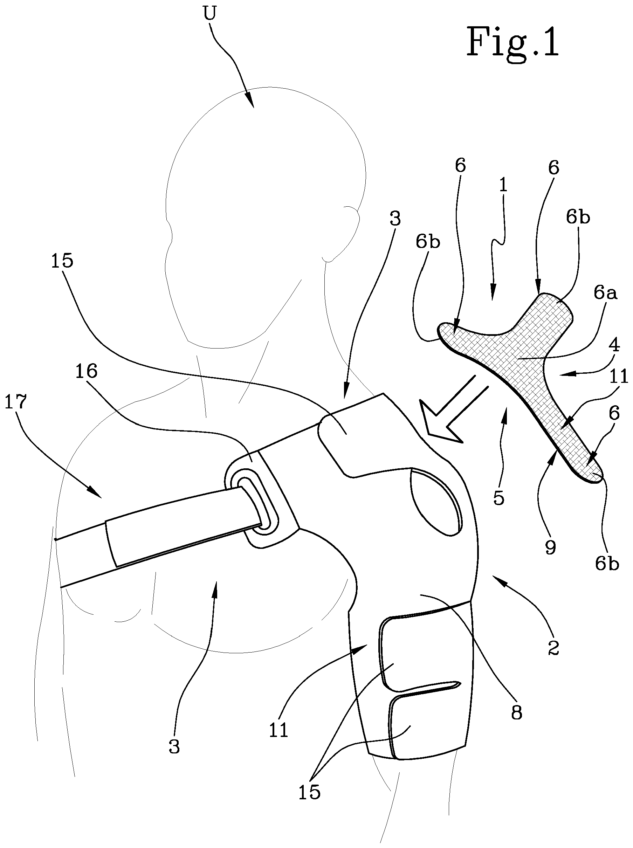

With reference to the appended figures 1 indicates overall an elastic brace according to the present invention.

It is specified that, preferably, the following description refers to a brace 1 adapted to be applied to the shoulder of a user "U" for the treatment of pathologies confined to the acromioclavicular joint.

However, the present invention may be applied to any joint in which a dual function is required, both for pathological treatment and for preservative action on the joint.

In particular, the elastic brace comprises a main body 2, made of flexible material and in the form of a sheet to be wrapped at least partially around a respective joint to be treated. As specified above, in the appended figures, a main portion 2 coupled to the shoulder of the user "U" is illustrated by way of non-limiting example.

The main body 2 is equipped with closing means 3 to be engaged in a stable way with the main body 2 itself on the joint.

Again, the brace 1 is also comprised of an anatomic interaction element 4 which can be associated with the main body 2 in a plurality of operating configurations, each of which corresponds to a respective mechanical action that the brace 1 performs on the joint.

In more detail, the interaction element 4 and the main body 2 are provided in two separate bodies that are coupled to one another reversibly so as to define a respective operating configuration. The coupling of the anatomic interaction element 4 onto the main body 2 takes place manually through appropriate coupling means 5 that will be described in more detail below.

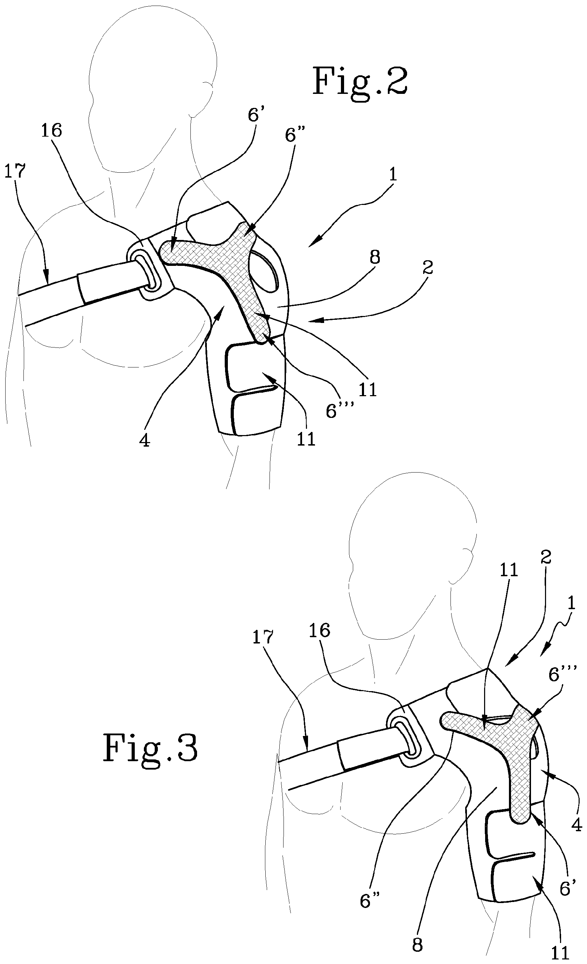

Advantageously, with reference to figures 2 and 3, the interaction element 4 can be switched between a first operating configuration (figure 2) wherein it actuates a joint stabilisation action adapted to prevent anterior dislocations and sub dislocations of the humeral head, and a second operating condition (figure 3) wherein it actuates a traction action of the limb to compensate for the dislocation of the humeral head.

For that purpose, the interaction element 4 in the first configuration (figure 2) is coupled to the main body 2 at a respective anterior zone to be wrapped around the acromioclavicular joint, part of the chest and the distal part of the deltoid.

In the second configuration (figure 3), the interaction element 4 is instead coupled to the main body 2 at a respective proximal zone to the deltoid symmetrically with respect to the coronal plane.

In this way, according to the positioning of the interaction element 4 on the main body 2 a respective mechanical action is actuated.

In fact, if the interaction element 4 is wrapped around the shoulder (figure 2) an immobilisation action is actuated, prevalently used for sports use or generally following anterior sub dislocation of the humeral head.

When, instead, the interaction element 4 is arranged laterally to the user "U" at the deltoid (second operating configuration of figure 3), the limb is retracted towards the shoulder to abduct the shoulder into the correct position defining a therapeutic action.

With reference to figure 6, note that the interaction element 4 comprises a plurality of end portions 6, extending reciprocally away from one another and each of which has an elongated conformation, defining a first common end 6a and a second distal end 6b from the first common end 6a.

In more detail, the interaction element 4 defines a substantially triangular shape wherein each angle is represented by the extension of the mentioned end portion. The centre of the triangle is instead defined by the mentioned first common end 6a.

In this situation, the interaction element 4 is defined by three end portions 6 equally spaced substantially at 120° from one another.

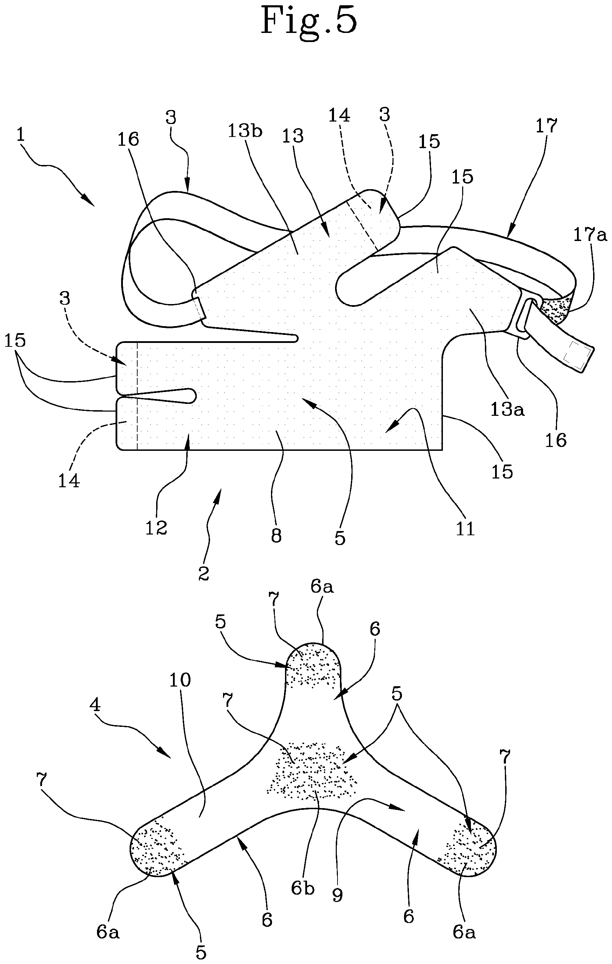

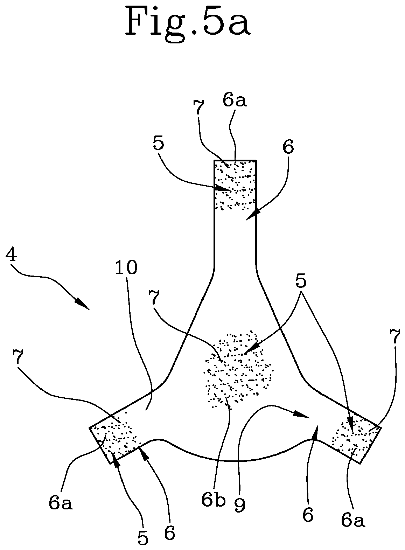

Note that the end portions 6 may have the same dimensions or could have different dimensions according to the specific function. For that purpose, as illustrated in figure 5, two end portions 6 are the same length, while a third portion 6 is shorter with respect to the first two.

On the other hand, in the alternative embodiment of figure 5a, two end portions 6 are the same length and are shorter than a third portion 6.

Still with reference to figure 5 and 5a, note that the reversible coupling means 5 comprise a plurality of zones 7 equipped with Velcro®, arranged respectively on an external visible surface 8 of the main body 2 and on each end 6a, 6b of the end portions 6.

For that purpose, the external surface 8 of the main body 2 is made of a fabric equipped with a plurality of slots that can be coupled to a plurality of hooks projecting from the ends 6a, 6b of each end portion 6. Advantageously, the interaction element 4 in the first configuration illustrated in figure 2 has: a first end portion 6' arranged at an upper zone of the chest and extending towards the breast bone of the user "U"; a second end portion 6" arranged at the collar bone and extending towards the supraspinatus muscle; and a third end portion 6'" arranged at the deltoid and extending towards the arm.

In this case, the first common end 6a of the end portions 6 is arranged on an anterior zone of the humeral head.

In the second configuration illustrated in figure 3, the interaction element 4 has: a first end portion 6' arranged along the humerus and having a respective second distal end 6b with respect to the deltoid; a second end portion 6" arranged at the front part of the torso and extending towards the chest; and a third end portion 6'" arranged at the rear part of the torso and extending towards the trapezium.

Note that the first common end 6a of the end portions 6 is arranged on the deltoid of the user "U".

The present invention can be used particularly with a respective material obtained through a multi-layer sheet. Both the main body 2 and the interaction element 4 are obtained from a multi-layer sheet that guarantees excellent elasticity and mechanical resistance characteristics.

In particular, the multi-layer sheet comprises an internal layer 9 made of fabric combined with carbon wire having an internal surface 10 to be in contact with the user's "U" skin. The multi-layer sheet further comprises an intermediate layer made of MTP foam (not shown in the appended figures) to allow the skin to breathe. Finally, the multi-layer sheet has an external layer 11 made of composite velcrable fabric with elastane.

Such external layer 11 of the main body 2 defines the mentioned external visible surface 8. In this situation, note that the hooks are afforded at the internal layer 9 of the anatomic interaction element 4.

With particular reference to figure 5, the main body 2 comprises a first portion 12 that can be wrapped around the user's humerus, and a second portion 13 that can be wrapped around the acromioclavicular joint.

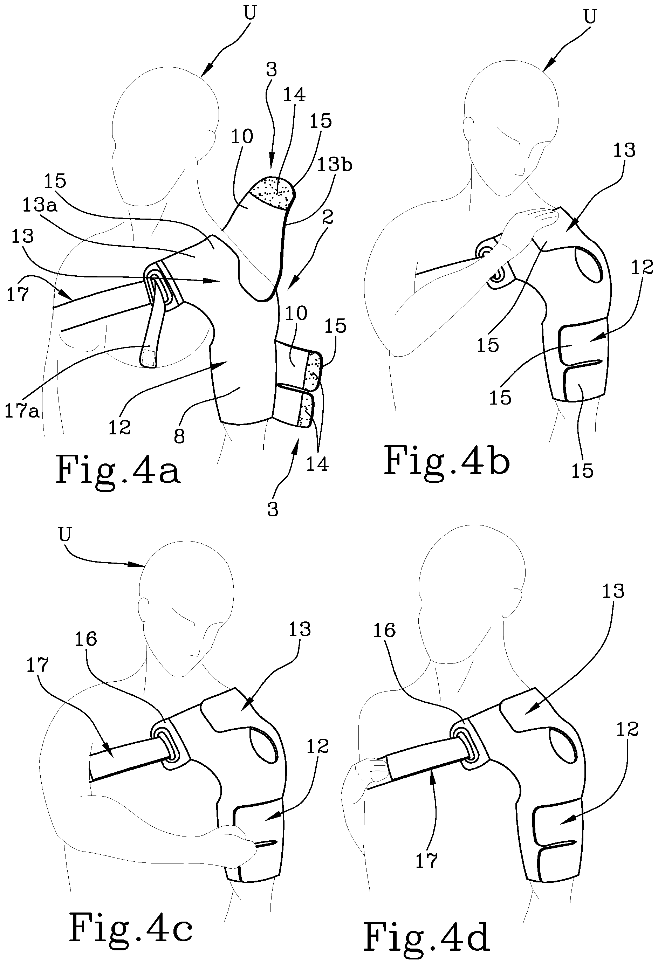

In particular, as illustrated in the view of figure 4a, the first portion 12 has a substantially rectangular conformation, to be wrapped in a "tubular" way around the humerus.

The second portion 13 is defined by two zones 13a, 13b, which can be moved towards one another to encircle the shoulder.

As shown in figures 4a and 4b, a first zone 13a is arranged on the anterior part of the shoulder while a second zone 13b is arranged on a posterior part of the shoulder.

Advantageously, the closing means 3 are comprised of a plurality of zones 14 equipped with Velcro® arranged on respective superimposable ends 15 of the first and second wrappable portion 12, 13.

The superimposable ends 15 of the first wrappable portion 12 are arranged at the biceps brachii (figures 4a-4d), while the superimposable ends 15 of the second wrappable portion 13 are arranged at the anterior deltoid (figures 4a-4d).

In this way, by superimposing the mentioned ends 15 it is possible to couple the main body 2 in a stable way to the user's "U" shoulder.

The second wrappable portion 13 further comprises respective secondary ends 16 opposite the superimposable ends 15. Such secondary ends have coupling zones to an elastic strip 17 which during use is arranged around the user's "U" torso.

The strip 17 is also equipped with a velcrable zone 17a applicable at the user's "U" chest to allow easy application and tightening of the whole brace 1 to the user's "U" body. Advantageously, a second end 16 is equipped with a slot into which the strip 17 passes and returns.

In this way, the user "U" manually tightens the elastic strip 17 around the torso, coupling it at the chest.

In more detail, with reference to the sequence shown in figures 4a-4d, note that the brace 1 is applicable by the actual user "U", only using their "free" hand, i.e. the hand of the arm not affected by the joint to be treated. Once the main portion 2 is worn (figure 4a), the velcroed ends 15 of the first and second wrappable portions 12, 13 (figure 4b) are superimposed. Following a first positioning, the relative position between the ends 15 is adjusted better (figure 4c) according to the mechanical action to be performed, the size of the limb and the comfort perceived by the user. Finally, the strip 17 is folded and closed onto itself by means of the return provided by the slot to actuate the stable locking of the main body 2 onto the torso and onto the user's "U" joint.

Once the main body 2 is associated, the user sees to the positioning of the anatomic interaction element 4 according to the action that the brace must perform on the joint.

Advantageously, the elastic brace 1 is still very versatile and able to actuate on the joint both a stabilisation and immobilisation action (used following dislocations), and a traction action of the arm to treat pathologies deriving from hemiplegia and/or hemiparesis which cause a gradual loss of motor activity.

In fact, according to the user's "U" requirements, the anatomic interaction element 4 is coupled to the main body 2 to actuate a specific action on the joint.

The coupling of the element 4 is also configurable according to the particular joint requirements and sizes, making the whole brace 1 particularly versatile and adaptable to any requirement, type and pathological severity.

The brace 1 is also structurally simple, inexpensive since it is able to actuate a series of actions on the joint, and easy to put on. On this point, note that the user "U" is able to put the brace 1 on independently and adjust it hence providing special tolerability and comfort for the brace 1 itself.

## Claims

1. An elastic brace (1) comprising:
- a main body (2), made of elastic material and that can be at least partially wrapped around a respective shoulder joint to be treated; and
- closing means (3) coupled to said main body (2) to firmly engage said main body (2) onto the joint;
- an anatomic interaction element (4) that is configured to be associated with said main body (2) in a plurality of operating configurations each of which corresponds to a respective mechanical action that the brace (1) performs on the joint;
said operating configurations comprising a first operating configuration wherein the interaction element (4) actuates a stabilisation action on the joint to prevent anterior dislocation and sub dislocation of the humeral head, and a second operating configuration in which the interaction element (4) actuates a traction action on the limb to compensate for the dislocation of the humeral head;
**characterised in that** said interaction element (4) comprises three end portions (6), extending mutually away from one another and each of which having an elongated conformation defining a first common end (6a) and a second distal end (6b) from said first common end (6a); the interaction element (4) defining a substantially triangular shape wherein each angle of said triangular shape is represented by the extension of an end portion (6) and the centre of said triangular shape is defined by the first common end (6a).

2. The brace according to the preceding claim, **characterised in that** said interaction element (4) is made of a separate body from said main body (2) and **in that** it further comprises reversible coupling means (5) between the interaction element (4) and the main body (2).

3. The brace according to claim 1, **characterised in that** said interaction element (4) in the first configuration is coupled to the main body (2) at a respective front zone to be wrapped around the acromioclavicular joint, part of the chest and the distal part of the deltoid.

4. The brace according to claim 3, **characterised in that** said interaction element (4) in the second configuration is coupled to the main body (2) at a respective proximal zone to the deltoid symmetrically with respect to the coronal plane.

5. The brace according claim 1, **characterised in that** the three end portions (6) are equidistant at 120° from one another.

6. The brace according to claim 2, **characterised in that** said reversible coupling means (5) comprise a plurality of zones (7) equipped with Velcro® arranged respectively on an external visible surface (8) of the main body (2) and on each end (6a, 6b) of said end portions (6).

7. The brace according to the preceding claim, **characterised in that** said external visible surface (8) of the main body (2) is made of a fabric equipped with a plurality of slots that can be coupled with a plurality of hooks projecting from the ends (6a, 6b) of each end portion (6).

8. The brace according to any one of claims 5 to 7, **characterised in that** said interaction element (4) is configured to have in the first configuration: a first end portion (6') arranged at an upper zone of the chest and extending towards the user's breast bone; a second end portion (6") arranged at the collarbone and extending towards the supraspinatus muscle; and a third end portion (6"') arranged at the deltoid and extending towards the arm; said first common end (6a) of the end portions (6', 6", 6"') being arranged on a front zone of the humeral head.

9. The brace according to any one of claims 5 to 8, **characterised in that** said interaction element (4) is configured to have the second configuration: a first end portion (6') arranged along the humerus and having a respective second distal end (6b) with respect to the deltoid; a second end portion (6") arranged at the front part of the torso and extending towards the chest; and a third end portion (6"') arranged at the rear part of the torso and extending towards the trapezium; said first common end (6a) of the end portions (6', 6", 6"') being arranged on the deltoid.

10. The brace according to any one of the preceding claims, **characterised in that** said main body (2) and said anatomic interaction element (4) are made of a multi-layer sheet comprising: an internal layer (9) made of fabric combined with carbon wire having an internal surface (10) to be in contact with the user's (U) skin; an intermediate layer made of MTP foam to allow the skin to breathe; and an external layer (11) made of composite velcrable fabric with elastane.

11. The brace according to claim 10 when it depends on claim 7, **characterised in that** the external layer (9) of the main body (2) defines said external visible surface (8) and **in that** said hooks project from the internal layer of the anatomic interaction element (4).

12. The brace according to any one of the preceding claims, **characterised in that** said main body (2) comprises a first portion (12) configured to be wrapped around the user's humerus, and a second portion (13) configured to be wrapped around the acromioclavicular joint.

13. The brace according to the preceding claim, **characterised in that** said closing means (3) have a plurality of zones (14) equipped with Velcro® arranged on respective superimposable ends (15) of said first and second wrappable portions (12, 13); said superimposable ends (15) of the first wrappable portion (12) being in use arranged at the biceps brachii, and said superimposable ends (15) of the second wrappable portion (13) being in use arranged at the anterior deltoid.

14. The brace according to the preceding claim, **characterised in that** said second wrappable portion (13) further comprises respective secondary ends (16) opposite the superimposable ends (15); and **in that** said main body (2) further comprises an elastic strip (17) engageable with said second ends (16).

15. The brace according to the preceding claim, **characterised in that** said elastic strip (17) is configured to be in use arranged around the user's torso and has closing Velcro (17a) applicable at the chest of the actual user.

## Patentansprüche

1. Elastische Orthese (1), umfassend:
- einen Hauptkörper (2), bestehend aus elastischem Material, der mindestens teilweise um ein jeweiliges zu behandelndes Schultergelenk gewickelt werden kann, und
- Verschlussmittel (3), gekuppelt mit dem Hauptkörper (2), um den Hauptkörper (2) fest auf dem Gelenk zu befestigen;
- ein anatomisches Interaktionselement (4), das ausgelegt ist, um mit dem Hauptkörper (2) in einer Vielzahl von Betriebskonfigurationen assoziiert zu werden,
von denen eine jede einem jeweiligen mechanischen Vorgang entspricht, den die Orthese (1) am Gelenk durchführt,
wobei diese Betriebskonfigurationen eine erste Betriebskonfiguration umfassen, in der das Interaktionselement (4) einen Stabilisierungsvorgang am Gelenk durchführt, um eine anteriore Dislokation und eine Subdislokation des Humeruskopfs zu vermeiden, und eine zweite Betriebskonfiguration, in der das Interaktionselement (4) einen Zugvorgang auf der Gliedmaße durchführt, um die Dislokation des Humeruskopfs auszugleichen,
**dadurch gekennzeichnet, dass** das Interaktionselement (4) drei Endabschnitte (6) umfasst, die sich gegenseitig wegführend voneinander erstrecken, und von denen ein jeder eine verlängerte Beschaffenheit aufweist, definierend ein erstes gemeinsames Ende (6a) und ein zweites distales Ende (6b) vom ersten gemeinsamen Ende (6a), wobei das Interaktionselement (4) eine im Wesentlichen dreieckige Form definiert, wobei ein jeder Winkel dieser dreieckigen Form durch die Ausdehnung eines Endabschnitts (6) dargestellt ist und die Mitte der dreieckigen Form durch das erste gemeinsame Ende (6a) definiert ist.

2. Orthese nach dem vorhergehenden Anspruch, **dadurch gekennzeichnet, dass** das Interaktionselement (4) aus einem separaten Körper vom Hauptkörper (2) besteht und dass es zudem reversible Kupplungsmittel (5) zwischen dem Interaktionselement (4) und dem Hauptkörper (2) umfasst.

3. Orthese nach Anspruch 1, **dadurch gekennzeichnet, dass** das Interaktionselement (4) in der ersten Konfiguration mit dem Hauptkörper (2) an einer jeweiligen frontseitigen Zone gekuppelt ist, die rund um das Acromio-Clavicular-Gelenk, einen Teil des Brustkorbs und den distalen Teil des Deltamuskels zu wickeln ist.

4. Orthese nach Anspruch 3, **dadurch gekennzeichnet, dass** das Interaktionselement (4) in der zweiten Konfiguration mit dem Hauptkörper (2) an einer jeweiligen proximalen Zone zum Deltamuskel gekoppelt ist, symmetrisch zur Frontalebene.

5. Orthese nach Anspruch 1, **dadurch gekennzeichnet, dass** die drei Endabschnitte (6) um 120° gleichabständig voneinander sind.

6. Orthese nach Anspruch 2, **dadurch gekennzeichnet, dass** die reversiblen Kupplungsmittel (5) eine Vielzahl an Zonen (7) umfassen, die mit Velcro® versehen sind, angeordnet jeweils auf einer außenseitigen sichtbaren Oberfläche (8) des Hauptkörpers (2) und an einem jeden Ende (6a, 6b) der Endabschnitte (6).

7. Orthese nach dem vorhergehenden Anspruch, **dadurch gekennzeichnet, dass** die außenseitige sichtbare Oberfläche (8) des Hauptkörpers (2) aus einem Gewebe besteht, versehen mit einer Vielzahl an Schlitzen, die mit einer Vielzahl an Haken gekuppelt werden können, die aus den Enden (6a, 6b) eines jeden Endabschnitts (6) hervorstehen.

8. Orthese nach einem der Ansprüche 5 bis 7, **dadurch gekennzeichnet, dass** das Interaktionselement (4) ausgelegt ist, um in der ersten Konfiguration einen ersten Endabschnitt (6') aufzuweisen, angeordnet an einer oberen Zone des Brustkorbs und sich erstreckend hinführend zum Brustknochen des Nutzers, einen zweiten Endabschnitt (6"), angeordnet am Schlüsselbein und sich erstreckend hinführend zum M. supraspinatus, und einen dritten Endabschnitt (6"'), angeordnet am Deltamuskel und sich erstreckend hinführend zum Arm, wobei das erste gemeinsame Ende (6a) der Endabschnitte (6', 6", 6"') an einer frontseitigen Zone des Humeruskopfs angeordnet ist.

9. Orthese nach einem der Ansprüche 5 bis 8, **dadurch gekennzeichnet, dass** das Interaktionselement (4) ausgelegt ist, um in der zweiten Konfiguration aufzuweisen: einen ersten Endabschnitt (6'), angeordnet entlang des Humerus und aufweisend ein jeweiliges zweites distales Ende (6b) im Vergleich zum Deltamuskel; einen zweiten Endabschnitt (6"), angeordnet am frontseitigen Teil des Rumpfs und sich erstreckend hinführend zum Brustkorb, und einen dritten Endabschnitt (6"'), angeordnet am rückseitigen Teil des Rumpfs und sich erstreckend hinführend zum Trapezmuskel, wobei das erste gemeinsame Ende (6a) der Endabschnitte (6', 6", 6"') am Deltamuskel angeordnet ist.

10. Orthese nach einem der vorhergehenden Ansprüche, **dadurch gekennzeichnet, dass** der Hauptkörper (2) und das anatomische Interaktionselement (4) aus einer mehrschichtigen Bahn bestehen, umfassend: eine innere Schicht (9), bestehend aus einem Gewebe, kombiniert mit Kohlenstoffdraht, aufweisend eine innenseitige Oberfläche (10), um mit der Haut des Nutzers (U) in Berührung zu sein; eine Zwischenschicht, bestehend aus MTP-Schaumstoff, um der Haut die Atmung zu erlauben, und eine äußere Schicht (11), bestehend aus einem klettbaren Verbundgewebe mit Elastan.

11. Orthese nach Anspruch 10, wobei dieser von Anspruch 7 abhängt, **dadurch gekennzeichnet, dass** die äußere Schicht (9) des Hauptkörpers (2) die außenseitige sichtbare Oberfläche (8) definiert und dass die Haken aus der inneren Schicht des anatomischen Interaktionselements (4) hervorstehen.

12. Orthese nach einem der vorhergehenden Ansprüche, **dadurch gekennzeichnet, dass** der Hauptkörper (2) einen ersten Abschnitt (12) umfasst, ausgelegt, um um den Humerus des Nutzers gewickelt zu werden, und einen zweiten Abschnitt (13), ausgelegt, um um das Acromio-Clavicular-Gelenk gewickelt zu werden.

13. Orthese nach dem vorhergehenden Anspruch, **dadurch gekennzeichnet, dass** die Verschlussmittel (3) eine Vielzahl an Zonen (14) aufweisen, die mit Velcro® ausgestattet sind, angeordnet auf jeweiligen übereinanderlagerbaren Enden (15) des ersten und zweiten wickelbaren Abschnitts (12, 13), wobei die übereinanderlagerbaren Enden (15) des ersten wickelbaren Abschnitts (12) in Verwendung am M. biceps brachii angeordnet sind und die übereinanderlagerbaren Enden (15) des zweiten wickelbaren Abschnitts (13) in Verwendung am vorderen Deltamuskel angeordnet sind.

14. Orthese nach dem vorhergehenden Anspruch, **dadurch gekennzeichnet, dass** der zweite wickelbare Abschnitt (13) zudem jeweilige Sekundärenden (16) umfasst, die gegenständig zu den übereinanderlagerbaren Enden (15) angeordnet sind, und dass der Hauptkörper (2) zudem einen elastischen Streifen (17) umfasst, der mit den Sekundärenden (16) in Eingriff gelangen kann.

15. Orthese nach dem vorhergehenden Anspruch, **dadurch gekennzeichnet, dass** der elastische Streifen (17) ausgelegt ist, um in Verwendung rund um den Rumpf des Nutzers angeordnet zu werden, sowie einen Klettverschluss (17a) aufweist, der am Brustkorb des effektiven Nutzers angebracht werden kann.

## Revendications

1. Orthèse élastique (1) comprenant :
- un corps principal (2) en matière plastique et pouvant être au moins partiellement enveloppé autour d'une articulation d'une épaule respective à soigner ; et
- des moyens de fermeture (3) accouplés au dit corps principal (2) pour fermement mettre en prise ledit corps principal (2) sur l'articulation ;
- un élément d'interaction anatomique (4) étant configuré pour être associé au dit corps principal (2) dans une pluralité de configurations fonctionnelles, chacune
correspondant à une action mécanique respective que l'orthèse (1) réalise sur l'articulation ;
lesdites configurations fonctionnelles comprenant une première configuration de fonctionnement dans laquelle l'élément d'interaction (4) active une action de stabilisation sur l'articulation pour empêcher une dislocation et une sous dislocation antérieure de la tête de l'humérus, et une seconde configuration de fonctionnement dans laquelle l'élément d'interaction (4) active une action de traction sur le membre pour compenser la dislocation de la tête de l'humérus ; **caractérisée en ce que** ledit élément d'interaction (4) comprend trois parties terminales (6) se prolongeant mutuellement en s'éloignant l'une de l'autre et chacune comportant une forme allongée définissant une première extrémité commune (6a) et une seconde extrémité distale (6b) à partir de ladite première extrémité commune (6a) ; l'élément d'interaction (4) définissant une forme substantiellement triangulaire dans laquelle chaque angle de ladite forme triangulaire est représenté par l'extension d'une partie terminale (6) et le centre de ladite forme triangulaire est défini par la première extrémité commune (6a).

2. Orthèse selon la revendication précédente, **caractérisée en ce que** ledit élément d'interaction (4) se compose d'un corps séparé dudit corps principal (2) et **en ce qu'**elle comprend de plus des moyens d'accouplement réversibles (5) entre l'élément d'interaction (4) et le corps principal (2).

3. Orthèse selon la revendication 1, **caractérisée en ce que** ledit élément d'interaction (4) dans la première configuration est accouplé au corps principal (2) en correspondance d'une zone antérieure respective à envelopper autour de l'articulation acromio-claviculaire, d'une partie de la poitrine et de la partie distale du deltoïde.

4. Orthèse selon la revendication 3, **caractérisée en ce que** ledit élément d'interaction (4) dans la seconde configuration est accouplé au corps principal (2) en correspondance d'une zone proximale sur le deltoïde de façon symétrique par rapport au plan frontal.

5. Orthèse selon la revendication 1, **caractérisée en ce que** les trois parties terminales (6) sont équidistantes à 120° les unes des autres.

6. Orthèse selon la revendication 2, **caractérisée en ce que** lesdits moyens d'accouplement réversibles (5) comprennent une pluralité de zones (7) équipées de Velcro® disposés respectivement sur une surface visible externe (8) du corps principal (2) et sur chaque extrémité (6a, 6b) desdites parties terminales (6).

7. Orthèse selon la revendication précédente, **caractérisée en ce que** ladite surface visible externe (8) du corps principal (2) est constituée d'un tissu pourvu d'une pluralité de fentes pouvant être accouplées à une pluralité de crochets dépassant des extrémités (6a, 6b) de chaque partie terminale (6).

8. Orthèse selon l'une quelconque des revendications de 5 à 7, **caractérisée en ce que** ledit élément d'interaction (4) est configuré pour comporter, dans la première configuration, une première partie finale (6') disposée en correspondance d'une zone supérieure de la poitrine et se prolongeant vers le sternum de l'utilisateur ; une seconde partie terminale (6") disposée en correspondance de la clavicule et se prolongeant vers le muscle supraépineux ; et une troisième partie terminale (6"') disposée en correspondance du deltoïde et se prolongeant vers le bras ; ladite première extrémité commune (6a) des parties terminales (6', 6", 6'") étant disposée sur une zone frontale de la tête de l'humérus.

9. Orthèse selon l'une quelconque des revendications de 5 à 8, **caractérisée en ce que** ledit élément d'interaction (4) est configuré pour comporter, dans la seconde configuration, une première partie finale (6') disposée le long de l'humérus et comportant une seconde extrémité distale (6b) respective par rapport au deltoïde ; une seconde partie terminale (6") disposée en correspondance de la partie antérieure du torse et se prolongeant vers la poitrine ; et une troisième partie terminale (6"') disposée en correspondance de la partie postérieure du torse et se prolongeant vers le trapèze ; ladite première extrémité commune (6a) des parties terminales (6', 6", 6'") étant disposée sur le deltoïde.

10. Orthèse selon l'une quelconque des revendications précédentes, **caractérisée en ce que** ledit corps principal (2) et ledit élément d'interaction anatomique (4) sont constitués d'une feuille multicouche comprenant : une couche interne (9) en tissu combiné à du fil carbone comportant une surface interne (10) se trouvant en contact avec la peau de l'utilisateur (U) ; une couche intermédiaire constituée de mousse MTP pour permettre à la peau de respirer ; et une couche externe (11) en tissu composite à velcros avec de l'élasthanne.

11. Orthèse selon la revendication 10 lorsqu'elle dépend de la revendication 7, **caractérisée en ce que** la couche externe (9) du corps principal (2) définit ladite surface visible externe (8) et **en ce que** lesdits crochets dépassent de la couche interne de l'élément d'interaction anatomique (4).

12. Orthèse selon l'une quelconque des revendications précédentes, **caractérisée en ce que** ledit corps principal (2) comprend une première partie (12) configurée pour être enveloppée autour de l'humérus de l'utilisateur et une seconde partie (13) configurée pour être enveloppée autour de l'articulation acromio-claviculaire.

13. Orthèse selon la revendication précédente, **caractérisée en ce que** lesdits moyens de fermeture (3) comportent une pluralité de zones (14) dotées de Velcro® disposés sur des extrémités superposables (15) respectives desdites première et seconde parties enveloppables (12, 13) ; lesdites parties superposables (15) de la première partie enveloppable (12) étant, lors de l'utilisation, disposées en correspondance du muscle biceps brachial, et lesdites extrémités superposables (15) de la seconde partie enveloppable (13) étant, lors de l'utilisation, disposées en correspondance du deltoïde antérieur.

14. Orthèse selon la revendication précédente, **caractérisée en ce que** ladite seconde partie enveloppable (13) comprend de plus des extrémités secondaires (16) respectives opposées aux extrémités superposables (15) ; et **en ce que** ledit corps principal (2) comprend de plus une bande élastique (17) se mettant en prise avec lesdites extrémités secondaires (16).

15. Orthèse selon la revendication précédente, **caractérisée en ce que** ladite bande élastique (17) est configurée pour être, lors de l'utilisation, disposée autour du torse de l'utilisateur et comporte un Velcro de fermeture (17a) applicable à la poitrine de l'utilisateur.
